# FASCICULE DE BREVET EUROPEEN

(11) **EP 4 344 405 B1**
(45) Date de publication et mention de la délivrance du brevet: **16.10.2024**
(21) Numéro de dépôt: 22728640.8
(22) Date de dépôt: 24.05.2022
(51) Int. Cl.: A61B 8/08, A61B 8/00

(54) **DISPOSITIF POUR L'ACQUISITION D'UNE SEQUENCE D'IMAGES ECHOGRAPHIQUES ET PROCEDE ASSOCIE**
VORRICHTUNG ZUR ERFASSUNG EINER SEQUENZ VON ECHOGRAFISCHEN BILDERN UND ZUGEHÖRIGES VERFAHREN
DEVICE FOR THE ACQUISITION OF A SEQUENCE OF ECHOGRAPHIC IMAGES AND ASSOCIATED METHOD

(30) Priorité: 28.05.2021 FR 2105597
(43) Date de publication de la demande: 03.04.2024
(73) Titulaire: Deski, 33000 Bordeaux (FR)
(72) Inventeur: MOAL, Olivier, 33300 Bordeaux (FR); MOAL, Bertrand, 33300 Bordeaux (FR); ROGER, Emilie, 33300 Bordeaux (FR)
(74) Mandataire: Oak & Fox
(86) Numéro de dépôt international: PCT/EP2022/064109
(87) Numéro de publication internationale: WO 2022/248500

(56) Documents cités:
- US-A1- 2019 130 554
- US-A1- 2020 245 976

## Description

### Domaine de l'invention

La présente invention concerne un dispositif et un procédé permettant d'automatiser et d'assister à un opérateur à la prise de vues échographiques. La présente invention permet avantageusement d'enregistrer automatiquement des prises de vues spécifiques du coeur permettant ultérieurement à un clinicien d'analyser ces dernières.

### État de la technique

L'imagerie échographique, aussi connue sous le terme d'imagerie à ultrason, est une technique d'imagerie médicale utilisant des ondes hautesfréquences pour visualiser une structure en deux dimensions à l'intérieur du corps d'un sujet vivant. Puisque les images échographiques sont prises en temps réel, ces images échographiques montrent le mouvement des organes internes dans le corps comme les mouvements du coeur lors de ces battements.

Pour acquérir de telles images, une sonde échographique est placée directement sur la peau du sujet. Une fine couche de gel peut être appliquée sur la peau pour permettre aux ondes ultrasonores de traverser la peau depuis la sonde jusqu'à l'intérieur du corps du sujet. Les images échographiques sont produites à partir de la mesure de la réflexion des ondes ultrasonores sur les organes du sujet. L'amplitude des ondes réfléchies mesurées, et le temps de vol de cette onde fournissent l'information nécessaire pour reconstruire l'image échographique.

Lors d'un examen échographique du coeur, l'opérateur doit savoir où placer la sonde et comment l'orienter pour obtenir l'image souhaitée. Généralement, les images souhaitées d'une échographie du coeur sont des images permettant de réaliser certaines mesures, notamment des longueurs d'organes ou de tissus.

Afin d'augmenter la capacité d'examen échographique, il a été pensé à soulager les cardiologues de la prise d'images. Par exemple, les images de vues peuvent être prises par un opérateur qui enregistrera les images puis les transmettra à un cardiologue qui n'aura alors plus qu'à les interpréter. Une telle organisation permet d'augmenter le nombre d'examens échographiques d'un cardiologue. Cependant, il faut alors être certains que les images échographiques sont de qualité suffisante pour leur interprétation par un cardiologue. L'opérateur peut avoir des connaissances en échographie, mais lorsqu'il n'effectuera pas lui-même l'analyse des images, il existe un besoin d'un dispositif d'assistance à la prise de telles images échographiques. De plus, l'opérateur ne doit pas juste acquérir une unique image de qualité suffisante, mais une séquence d'images suffisamment longue, par exemple sur un cycle de battement complet pour que le cardiologue puisse réaliser son diagnostic et/ou on analyse.

On connait des méthodes implémentées par logiciel pour guider l'opérateur à la prise d'images échographiques du coeur. Ces méthodes comprennent l'évaluation de la qualité d'une séquence d'images par rapport à une vue particulière et l'enregistrement d'une telle séquence.

Le document US 2019/130554 A1 divulgue un procédé permettant d'effectuer des mesures automatiques sur une séquence d'images recueillies par un dispositif ultrasonore. Ce procédé consiste à calculer la qualité de la séquence d'images recueillies par un dispositif ultrasonore pendant l'imagerie à l'aide d'un dispositif informatique.

Un inconvénient de ces méthodes est qu'il reste difficile pour l'opérateur d'évaluer quand la qualité de l'image qu'il est un train de prendre est suffisante. Un autre inconvénient de ces méthodes est qu'il peut être difficile d'obtenir une séquence d'images suffisamment longue correspondant à la qualité exigée, rendant d'autant plus la tâche de l'opérateur qui ne verra pas sa prise de vue validée par le logiciel.

Il existe donc un besoin d'un nouveau dispositif permettant de faciliter l'opérateur dans sa prise de vues échographiques.

L'invention vise donc à fournir un dispositif et un procédé associé permettant à des opérateurs d'acquérir plus facilement et plus rapidement des séquences d'images échographiques de qualité suffisante.

### Résumé de l'invention

Selon un aspect, l'invention concerne un dispositif d'assistance à la création ou l'acquisition d'une séquence d'images échographiques, préférentiellement d'images échographiques du coeur, configuré pour recevoir en temps réel des images échographiques acquises par une sonde échographique connectée audit dispositif, et comprenant :
- un premier calculateur, associé à une première mémoire, pour mettre en oeuvre un classifieur configuré pour classifier en temps réel les images échographiques reçues et associer à chaque image une classe pour générer un indicateur de qualité en fonction de ladite classe associée ;
- un afficheur pour, en temps réel, afficher les images échographiques reçues et pour afficher le dernier indicateur de qualité généré ;
- un second calculateur, associé à une seconde mémoire, et configuré pour exécuter les étapes suivantes :
   ∘ lorsqu'une séquence d'un nombre prédéfini d'images reçues comprend un taux d'images associées à une même classe supérieur à un taux prédéfini, l'enregistrement automatiquement de ladite séquence d'images dans une troisième mémoire ;
   ∘ la génération et l'affichage sur l'afficheur en temps réel d'un indicateur temporel représentant le nombre d'images restantes à recevoir pour atteindre ladite séquence d'un nombre prédéfini d'images reçues comprenant un taux d'images associées à une même classe supérieur à un taux prédéfini.

Un avantage est de générer une image en temps réel comprenant un indicateur de qualité indiquant à l'opérateur que l'image acquise est de qualité suffisante et l'indicateur temporel indiquant à l'opérateur que l'acquisition d'une séquence vidéo est en cours ainsi que le temps restant durant lequel il doit conserver une position dans laquelle les images acquises sont toujours de qualité suffisante. Les séquences ainsi enregistrées permettent de générer des séquences vidéo que le cardiologue pourra utiliser pour établir son diagnostic.

Selon un mode de réalisation, la première, la seconde et la troisième mémoire sont une même mémoire. Selon un mode de réalisation, la première et la seconde mémoire sont une même mémoire. Selon un mode de réalisation, la première et la troisième mémoire sont une même mémoire. Selon un mode de réalisation, la seconde et la troisième mémoire sont une même mémoire. Ainsi, dans la présente demande, on désigne indifféremment une mémoire comme un dispositif physique pour stocker des données ou un espace mémoire disponible d'un dispositif physique.

Selon un mode de réalisation, le premier calculateur et/ou le second calculateur est/sont compris localement dans le dispositif de l'invention. Selon un autre mode, le premier calculateur et/ou le second calculateur est/sont compris dans un équipement distant tel qu'un serveur de données. Dans ce dernier cas, le dispositif de l'invention s'entend comme un système comportant plusieurs équipements.

Dans un mode de réalisation, le classifieur est configuré pour classifier une image parmi une pluralité de classes, représentant chacune une vue particulière de l'organe du coeur, et au moins une classe représentant une vue de qualité insuffisante. L'avantage est de permettre de détecter aussi bien une vue particulière du coeur que des images qui ne seraient pas utilisables par le cardiologue pour établir un diagnostic.

Dans un mode de réalisation, le classifieur est mis en oeuvre au moyen d'une fonction apprenante configurée à partir d'un apprentissage automatique supervisé.

Dans un mode de réalisation, le classifieur est mis en oeuvre au moyen d'un réseau de neurones entrainé par une série d'images échographiques du coeur labélisées. L'avantage d'un tel classifieur est qu'il permet une classification fiable des images échographiques à partir d'un entrainement à partir d'images labélisées.

Selon un exemple, le réseau de neurones est un réseau neuronal convolutif, dit CNN ou Convet.

Selon un mode de réalisation, la configuration du réseau de neurones CNN peut comprendre :
▪ Des convolutions ou des couches de réseau de neurones comportant une pluralité de multiplications de matrices comportant des coefficients de pondération obtenus à partir d'un procédé d'apprentissage ;
▪ des opérations non-linéaires.

Selon un mode de réalisation, la configuration du réseau de neurones CNN comprend en entrée(s) des images acquises ou reçues ou stockées dans une mémoire.

Le réseau de neurones CNN peut comprendre dans ses premières couches des convolutions puis des couches de neurones entièrement connectées, dite « fully connected layers » à la fin du modèle. Dans ce dernier cas, ce sont des neurones connectés à tous les neurones de la couche précédente et connectés à tous ceux de la couche suivante.

Les couches de convolutions peuvent comprendre un balayage d'une matrice d'entrée produisant une série de calculs matriciels. Les autres couches du réseau de neurones comprennent, elles généralement des calculs matriciels sur la taille de la matrice d'entrée.

Selon un exemple, chaque convolution comprend un produit de matrice entre une matrice d'entrées et une matrice de poids et la prise en compte d'un biais additionnel.

L'application d'un traitement par couches successives au sein du réseau de neurones CNN comprend l'application d'une série de multiplications matricielles qui sont suivies d'une fonction non linéaire pour produire une sortie de ladite couche. La succession de ces opérations définit la profondeur du réseau de neurones.

Selon un exemple de réalisation, le réseau de neurones est un perceptron multicouche, connu sous l'acronyme MLP et dans la terminologie anglo-saxonne par « multi-layers perceptron ». Selon un exemple, le réseau de neurones peut être un réseau équivalent au MLP.

Dans un mode de réalisation, lesdits labels comportant une information caractéristique d'une prise de vue d'une image d'un organe comportant un angle de vue et/ou une taille caractéristique ; d'une qualité d'image ; de la présence d'une portion d'une image du coeur et /ou de la présence d'un contour de forme anatomique mesurable.

Dans un mode de réalisation, l'indicateur de qualité affiché comprend un indicateur coloré dont la couleur dépend de la classe associée à la dernière image classifiée. Dans un mode de réalisation, une couleur est associée aux images échographiques classifiées comme étant de qualité insuffisante. L'avantage est de permettre à l'opérateur de rapidement visualiser si l'image qu'il acquiert est de qualité suffisante.

Dans un mode de réalisation, l'indicateur de qualité est fonction d'une valeur de confiance de la classification de la dernière image classifiée, générée par la classification de ladite image échographique. Dans un mode de réalisation, au moins une des dimensions de l'indicateur coloré affiché dépend de ladite valeur de confiance. Un avantage est de permettre à l'opérateur de visualiser, lorsque l'image qu'il acquiert est de bonne qualité, de visualiser si un mouvement de la sonde améliore la qualité ou rapproche l'image d'une image d'une qualité insuffisante.

Dans un mode de réalisation, l'indicateur temporel affiché comprend une jauge dont le remplissage dépend de la valeur de l'indicateur temporel. L'opérateur visualise avantageusement le temps restant durant lequel il doit maintenir une qualité d'acquisition suffisante pour la validation ou l'enregistrement automatique de la séquence.

Selon un autre aspect, l'invention concerne un système comprenant un dispositif selon l'invention et une sonde échographique. La sonde échographique est préférentiellement apte à être connectée audit dispositif de manière à transmettre les images échographiques acquises par la sonde au dispositif.

Selon un autre aspect, l'invention concerne un procédé d'assistance à la génération d'une séquence d'images échographiques, préférentiellement d'une séquence d'images échographiques du coeur mise en oeuvre par ordinateur.

Ledit procédé comprend :
▪ la réception et l'affichage en continu d'images échographiques, préférentiellement d'images échographiques du coeur depuis une sonde échographique ;
▪ la classification (200) en temps réel des images échographiques reçues, et l'association à chaque image d'une classe ;
▪ la génération et l'affichage d'un indicateur de qualité en fonction de ladite classe associée à l'image échographique classifiée ;
▪ lorsqu'une séquence d'un nombre prédéfini d'images reçues comprend un taux d'images associées à une même classe supérieur à un taux prédéfini, l'enregistrement automatiquement de ladite séquence d'images dans une mémoire ;
▪ la génération et l'affichage en temps réel d'un indicateur temporel représentant le nombre d'images restantes à recevoir pour atteindre ladite séquence d'un nombre prédéfini d'images reçues comprenant un taux d'images associées à une même classe supérieur à un taux prédéfini.

Dans un mode d'exécution, le procédé comprend en outre la sélection d'une première classe représentant une première vue particulière du coeur, ladite sélection générant automatiquement :
- l'affichage d'une première image préenregistrée correspondant à une image de la première vue échographique du coeur ladite première classe sélectionnée, et/ou
- l'affichage d'une première consigne préenregistrée de position de sonde échographique sur un patient pour capter ladite première vue particulière du coeur.

L'affichage de l'image et de la consigne préenregistrées permet de guider l'opérateur dans la recherche des images échographique à obtenir.

Dans un mode d'exécution, le procédé comprend en outre, après l'enregistrement automatique de ladite séquence automatique :
• la sélection automatique d'une seconde classe représentant une seconde vue particulière du coeur,
• L'affichage sur l'afficheur d'une seconde image échographique préenregistrée correspondant à ladite seconde classe sélectionnée, et/ou
• L'affichage sur l'afficheur d'une seconde consigne préenregistrée de position de sonde échographique sur un patient pour capter ladite vue seconde particulière du coeur.

Dans un mode d'exécution, une fraction prédéfinie des images échographiques reçues sont classifiées. Préférentiellement, ladite fraction prédéfinie est paramétrable ou pilotable par une interface utilisateur. Cette sélection du taux d'images reçues à classifier permet de toujours classifier les images reçues en temps réel, notamment lorsque la vitesse d'acquisition d'images échographiques est supérieure à la vitesse de classification de ces images. Dans un autre mode d'exécution, la classification est effectuée avec la dernière image échographique reçue par le récepteur REC.

Dans un mode d'exécution, l'étape de génération d'un indicateur de qualité comprend en outre la génération et l'affichage d'une valeur de confiance représentative de la certitude de la classification effectuée, et optionnellement dans lequel l'indicateur de qualité affiché comprend un indicateur coloré dont la couleur dépend de la classification de l'image échographique et dont au moins une dimension est fonction de la valeur de confiance.

Selon un autre aspect, l'invention concerne un dispositif comprenant des moyens logiciels et matériels pour exécuter le procédé selon l'invention. Les moyens matériels peuvent comprendre un afficheur, un récepteur REC, une sonde échographique, un ou plusieurs processeurs ou calculateurs associés à des mémoires, une ou plusieurs mémoires pour stocker les séquences d'images échographiques, et/ou un émetteur.

Selon un autre aspect, l'invention concerne un produit programme d'ordinateur comprenant des instructions qui conduisent le dispositif selon l'invention à exécuter les étapes du procédé selon l'invention. Selon un aspect, l'invention concerne un support lisible par ordinateur, sur lequel est enregistré le programme d'ordinateur selon l'invention.

### Brève description des figures

D'autres caractéristiques et avantages de l'invention ressortiront à la lecture de la description détaillée qui suit, en référence aux figures annexées, qui illustrent :
Fig. 1 : une vue schématique de l'image affichée par l'afficheur du dispositif selon un premier mode de réalisation de l'invention.
Fig. 2 : une vue schématique d'un dispositif selon un mode de réalisation de l'invention.
Fig. 3 : une vue schématique de l'image affichée par l'afficheur du dispositif selon un second mode de réalisation de l'invention.
Fig. 4 : un logigramme représentant les différentes étapes d'un procédé selon un mode d'exécution de l'invention.

### Description détaillée

L'invention concerne un procédé 1000 d'assistance à la génération d'une séquence d'images échographiques. L'invention est particulièrement avantageuse pour la génération d'une séquence d'images échographiques du coeur. En effet, le coeur est en organe soumis à un cycle.

Le cycle cardiaque se compose de deux périodes : une pendant laquelle le muscle cardiaque se détend et se remplit de sang, appelée diastole, suivie d'une période de contraction vigoureuse et le pompage du sang, appelé systole. Après s'être vidé, le coeur se détend immédiatement et se dilate pour recevoir un autre afflux de sang revenant des poumons et d'autres systèmes du corps, avant de se contracter à nouveau pour pomper le sang vers les poumons et ces systèmes. Un coeur qui fonctionne normalement doit être complètement dilaté avant de pouvoir pomper à nouveau efficacement.

Pour analyser d'éventuelles pathologies, il est important pour le praticien d'obtenir une séquence d'images (ou une vidéo) de l'intégralité du cycle cardiaque. L'invention permet avantageusement d'assister le praticien dans l'acquisition d'une séquence d'images.

Cependant, l'invention peut également trouver des avantages dans l'acquisition échographique d'autres organes ou une séquence d'images est souhaitable, par exemple dans le domaine de l'échographie obstétricale.

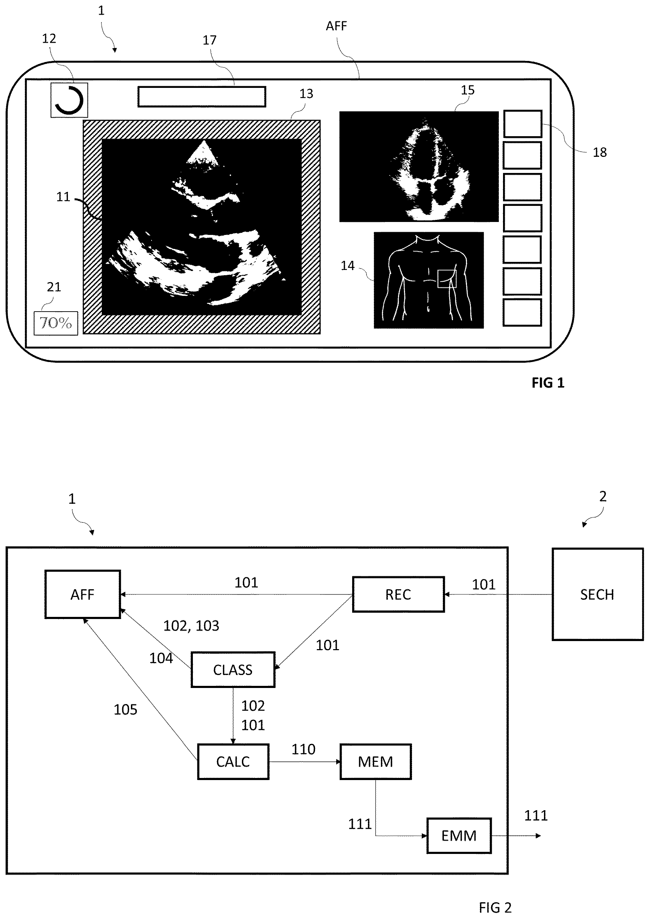

L'invention concerne également un dispositif associé. Un exemple d'un tel dispositif 1 est illustré sur la figure 2.

Le dispositif 1 peut comprendre une tablette, un smartphone, un ordinateur, ou tout autre dispositif comprenant au moins un afficheur et un processeur associé à une mémoire. Selon un autre aspect, l'invention concerne également un système 2 comprenant un dispositif 1 selon l'invention et une sonde échographique SECH connectée audit dispositif 1.

Dans un mode de réalisation, le dispositif comprend des moyens logiciels et matériels pour mettre en oeuvre le procédé selon l'invention décrit ci-après. Ledit dispositif comprend préférentiellement au moins un récepteur REC, un classifieur et un afficheur.

### RECEPTION

Le procédé comprend une étape de réception 100 d'images échographiques 11 en temps réel. Lesdites images échographiques sont reçues par un récepteur REC du dispositif. Dans un mode de réalisation, le récepteur REC peut comprendre une mémoire tampon dans laquelle sont stockées temporairement les images reçues 11 par le récepteur REC avant leur transmission.

Les images échographiques reçues 11 sont affichées en temps réel, préférentiellement sur un afficheur AFF du dispositif. Les images échographiques reçues peuvent donc être transmises à un afficheur pour leur affichage en temps réel.

Le dispositif comprend un afficheur AFF. L'afficheur AFF peut comprendre un écran tel l'écran d'un moniteur, d'une tablette tactile ou d'un téléphone intelligent.

L'afficheur AFF est connecté au récepteur REC et/ou au classifieur CLASS et/ou au processeur. L'afficheur AFF est configuré pour afficher en temps réel les images échographiques reçues par le récepteur REC. L'afficheur AFF permet avantageusement à l'opérateur d'avoir un retour en temps réel sur les images échographiques qu'il est en train d'acquérir. L'afficheur AFF est configuré pour afficher des indicateurs ou des données supplémentaires qui seront décrites plus loin dans la présente description. L'afficheur AFF affiche une image 1 comprenant une image échographique reçue 11 par le récepteur REC. Préférentiellement, l'image échographique affichée est la dernière image échographique reçue par le récepteur REC. L'afficheur AFF est ainsi configuré pour afficher en temps réel les images captées par la sonde échographique SECH connectée au dispositif selon l'invention.

### CLASSIFICATION

Le procédé comprend une étape de classification 200 des images échographiques 11 reçues par le récepteur REC en temps réel. La classification est réalisée par un classifieur CLASS du dispositif.

La classification d'une image échographique comprend l'association de ladite image échographique à une classe 14, préférentiellement une classe parmi un groupe de classe prédéfinie.

Dans un mode d'exécution, la classification comprend en outre la génération 300 d'un indicateur de qualité 13 en fonction de ladite classe associée à l'image échographique. L'indicateur de qualité peut être représentatif d'une classe ou d'un ensemble prédéfini de classes.

Dans un mode d'exécution, l'étape de classification comprend en outre la génération d'une valeur de confiance. La valeur de confiance peut représenter le taux de confiance de la classification réalisée. La valeur de confiance est générée par le classifieur CLASS.

L'étape de classification est préférentiellement exécutée par un classifieur CLASS du dispositif. Par classifieur, on entend une fonction algorithmique exécutée par un processeur ou un calculateur associé à une mémoire.

Le classifieur CLASS est configuré pour recevoir des images reçues par le récepteur REC.

Le classifieur est configuré pour classifier en temps réel au moins une partie des images reçues par le récepteur REC. Le classifieur est configuré pour associer à une image échographique une classe. Le classifieur génère un indicateur de qualité 13 en fonction de la classe associée. L'indicateur de qualité 13 est préférentiellement associé à ladite image échographique classifiée.

Préférentiellement, le dispositif des moyens de traitement d'image pour traiter les images reçues avant de les fournir au classifieur CLASS. Ces moyens de traitement peuvent comprendre des filtres d'images ou des fonctions de contraste.

Préférentiellement, le classifieur CLASS est configuré pour classifier une image échographique du coeur parmi une pluralité de classes comprenant, d'une part, des classes représentant chacune une vue particulière du coeur et d'autre part, une classe représentant une vue de qualité insuffisante.

Ainsi, lorsqu'une image est classifiée dans une classe représentant une vue particulière du coeur, cette image est considérée comme d'une qualité suffisante pour permettre avantageusement au clinicien de réaliser des mesures prédéfinies à partir de ces images. En effet, les cardiologues utilisent pour leurs diagnostics des vues particulières bien identifiées.

Dans un mode de réalisation, chaque classe représentant une vue particulière du coeur représente une vue parmi :
- La vue d'une coupe parasternale grand axe
- La vue d'une coupe parasternale petit axe
- La vue d'une coupe apicale
- La vue d'une coupe sous-costale
- La vue d'une coupe suprasternale
- La vue d'une coupe paraphernale droite

Chacune de ces vues est bien connue des cardiologues et est utilisée afin de visualiser différentes parties du coeur, calculer ou mesurer des données spécifiques, et identifier certaines pathologies.

Chacune de ces vues peut être caractérisé par la présence d'une ou plusieurs parties spécifiques du coeur. Par exemple, la vue d'une coupe parasternale petit axe est caractérisée par la présence sur l'image de la section du ventricule gauche et du ventricule droit. À partir de cette vue, le cardiologue peut calculer la fraction de raccourcissement et calculer des pressions pulmonaires.

Dans un mode de réalisation, la classe représentant une vue de qualité insuffisante représente des vues échographiques du coeur n'appartenant pas à une des vues spécifiques des autres classes ou des vues échographiques spécifiques listées ci-dessus, mais dont la qualité de l'image ne permet pas d'opérer les mesures nécessaires liées à cette vue spécifique.

Dans un mode de réalisation, le classifieur CLASS est mis en oeuvre au moyen d'une fonction apprenante entrainée à partir d'un apprentissage supervisé et/ou automatique. La fonction apprenante comprend préférentiellement un réseau de neurones. La fonction apprenante est préférentiellement entrainée par une série d'images échographiques du coeur labélisées.

Dans un exemple, la fonction apprenante a été configurée à partir d'un apprentissage comprenant la soumission au classifieur d'une pluralité d'images échographiques chacune associée à un label. Dans un mode de réalisation, le classifieur a été entrainé avec des images échographiques d'une vue spécifique du coeur de qualité suffisante, chaque image étant associée à un label représentant ladite vue spécifique. Le classifieur a également été entrainé avec des images échographiques de vues du coeur autre que celles citées ci-dessus ou ne présentant pas une qualité suffisante permettant au cardiologue d'effectuer des mesures à partir de ces images, chacune de ces images étant chacune associées à un label de qualité représentant une vue d'une qualité insuffisante. Les labels peuvent également comporter une information caractéristique d'une prise de vue d'une image d'un organe comportant un angle de vue et/ou une taille caractéristique, d'une qualité d'image, de la présence d'une portion spécifique d'une image du coeur, de la présence d'un contour de forme anatomique mesurable. Préférentiellement, les labels comprennent le nom de la vue particulière du coeur ou un nom associé à une vue de mauvaise qualité.

Le classifieur CLASS peut être exécuté dans un processeur lui-même associé à une mémoire. Le classifieur CLASS peut être enregistré dans un support lisible par ordinateur tel une mémoire associée audit processeur.

Dans un mode d'exécution, l'étape de classification d'une image échographique comprend la génération d'un score de correspondance pour chaque classe. Le score de correspondance peut comprendre une probabilité que l'image échographique appartienne à chaque classe. Le procédé comprend alors la sélection de la classe dont le score est le plus élevé. Préférentiellement, la valeur de confiance est générée à partir dudit score de correspondance de la classe associée à ladite image échographique.

Dans un mode de réalisation, le classifieur CLASS classifie 100% des images reçues en temps réel.

Dans un mode de réalisation alternatif, le classifieur CLASS est configuré pour classifier uniquement un taux d'images reçues. Le classifieur est alors configuré pour classifier une partie des images échographiques reçues en temps réel. Ce mode de réalisation est particulièrement avantageux lorsque la vitesse de classification est inférieure à la vitesse d'acquisition d'images (en images par unité de temps).

Dans un mode d'exécution, la classification d'une image échographique comprend la classification de la dernière image échographique reçue. Lorsque ladite image a été classifiée, le procédé prend à nouveau la dernière image échographique reçue. Ainsi, il est possible que certaines images échographiques ne soient pas classifiées. Cependant, ce mode d'exécution permet avantageusement de classifier en temps réel les images échographiques, peu importe la rapidité du classifieur ou la fréquence de réception des images échographiques.

### INDICATEUR DE QUALITE

Le procédé selon l'invention comprend la génération et l'affichage en temps réel sur l'afficheur AFF de l'indicateur de qualité 13 générée. L'indicateur de qualité 13 affiché correspond au dernier indicateur de qualité 13 généré.

Dans un mode d'exécution, cet indicateur de qualité 13 est généré par le classifieur CLASS. Un tel classifieur est configuré pour, lorsqu'il reçoit une image échographique du coeur, classifier ladite image dans l'une des classes mentionnées ci-dessus. Le procédé comprend alors la génération d'un indicateur de qualité représentatif de la classification de cette image. Ledit indicateur de qualité peut être associé à ladite image échographique classifiée.

De manière alternative, l'indicateur de qualité 13 peut être généré par un processeur distant connecté à l'afficheur AFF et recevant des informations du classifieur.

L'affichage de l'indicateur de qualité 13 peut comprendre l'affichage d'un indicateur coloré dont la couleur dépend de la classe associée à l'image échographique classifiée. La couleur de l'indicateur peut être représentative d'une unique classe. Préférentiellement, la couleur de l'indicateur coloré peut prendre deux couleurs distinctes, une première couleur représentative d'un groupe de classe incluant les classes représentant une vue particulière du coeur telle que celles énoncées ci-avant, et une seconde couleur représentative d'une classe représentant une vue de qualité insuffisante. Ainsi, l'opérateur visualise avantageusement plus rapidement si l'image échographique qu'il est en train de prendre est de qualité suffisante ou pas.

Dans un mode de réalisation, le classifieur est également configuré pour, lorsqu'il reçoit une image échographique du coeur, générer une valeur de confiance 21. La valeur de confiance 21 peut être représentative d'un niveau de certitude qui l'image a été bien classifiée. Dans un mode de réalisation, l'indicateur de qualité peut comprendre ladite valeur de confiance. L'indicateur de confiance 21 est préférentiellement affichée par l'afficheur AFF.

L'indicateur de qualité 13 associé à l'image classifiée peut inclure ledit indicateur de confiance. Dans un autre mode alternatif, un indicateur de confiance représentatif de la valeur de confiance est généré et affiché en temps réel.

Dans un premier exemple illustré sur la figure 1, l'indicateur de qualité 13 affiché comprend un cadre coloré, qui s'étendant préférentiellement autour de l'image échographique affichée. L'opérateur peut alors avantageusement visualiser si l'image qu'il prend est bien celle attendue sans avoir à détourner le regard de l'image. L'indicateur de confiance peut être affiché sous la forme d'une valeur numérique comme illustré sur la figure 1.

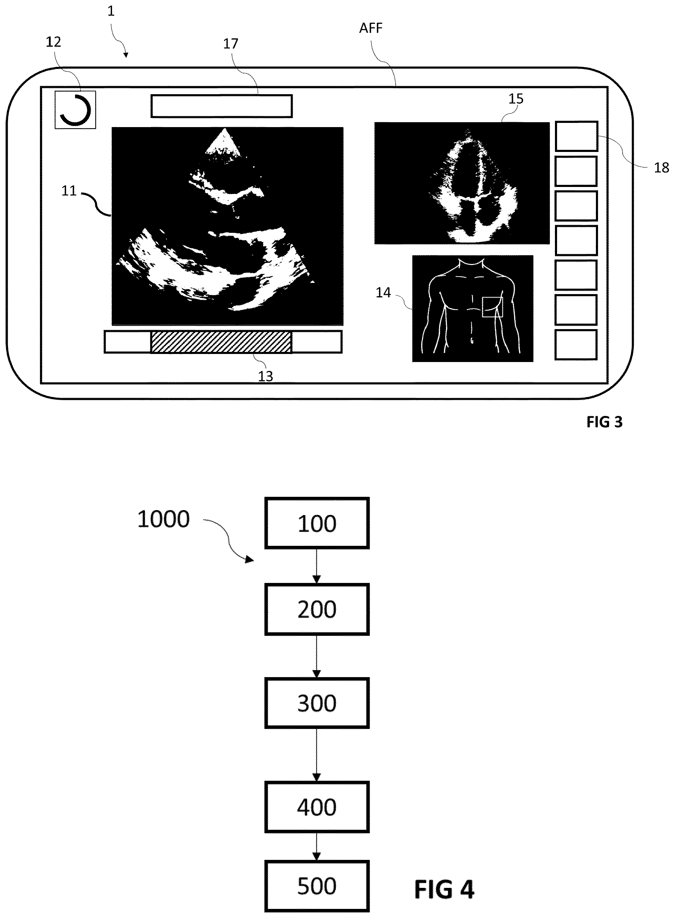

Dans un second exemple illustré sur la figure 3, l'indicateur de qualité 13 comprend un indicateur coloré. La couleur de l'indicateur coloré est représentative de la classification de la dernière image échographique. Au moins une des dimensions dudit indicateur coloré est fonction de la valeur de confiance. Sur l'exemple illustré sur la figure 3, l'indicateur de qualité 13 est une barre dont la longueur varie selon la valeur de confiance et dont la couleur dépend de la classe associée à la dernière image échographique.

Un tel indicateur permet avantageusement à l'opérateur de visualiser si l'image qu'il prend est de qualité suffisante et lui permet également de visualiser sans détourner le regard si l'indicateur est dégradé ou amélioré en fonction du mouvement de la sonde échographique SECH. L'intérêt d'un tel indicateur est de permettre à l'opérateur, lorsqu'il bouge légèrement la sonde, de visualiser si ce mouvement augmente ou réduit la valeur de confiance, et par conséquent augmente ou réduis ses chances d'obtenir une séquence d'images validée décrite ci-après.

### SEQUENCE

Le procédé comprend l'enregistrement automatique 400 d'une séquence 20 d'images dans une mémoire MEM. Cette séquence 20 est enregistrée automatiquement lorsqu'une séquence d'un nombre prédéfinie d'images reçues ou classifiées comprend un taux d'images associées à une même classe 14 supérieur à un taux prédéfini. Préférentiellement, ladite classe est une classe représentant une vue particulière du coeur.

Par « séquence d'images », on entend une série d'images échographiques qui se suivent selon l'ordre chronologique de leur acquisition. On utilisera donc aussi bien le terme « séquence vidéo » pour désigner une telle séquence d'images.

Ainsi, lorsqu'une vidéo suffisamment longue comprend, par exemple, une majorité d'images échographiques classifiées dans une même classe représentant une vue particulière du coeur, la séquence 20 vidéo est automatiquement enregistrée dans une mémoire. Cet enregistrement automatique génère avantageusement de manière automatique des vidéos d'une vue particulière du coeur qui pourra être analysée par un cardiologue sans validation de l'opérateur.

Le nombre prédéfini d'images reçues ou classifiées peut être compris comme une durée minimale prédéfinie, dans la mesure où la fréquence d'acquisition d'images de la sonde échographique SECH est constante. L'avantage de ce seuil est de s'assurer que la séquence 20 vidéo est suffisamment longue pour pouvoir être analysée par un cardiologue. Dans un autre mode d'exécution, le nombre prédéfini d'images peut être remplacé par un nombre d'images reçues ou classifiées. Le nombre prédéfini peut être paramétré pour correspondre à un nombre prédéfini de cycles cardiaques du coeur du sujet.

Le taux d'images associées à une même classe dans ladite séquence vidéo supérieur à un taux prédéfini permet avantageusement l'enregistrement automatique malgré un nombre négligeable d'images n'ayant pas une qualité suffisante dans ladite séquence. En effet, ce nombre négligeable d'images dans une séquence vidéo peut venir d'une erreur de classification, ou du bruit lié à une image particulière. Cette tolérance consiste avantageusement en un bon compromis entre la facilité de génération automatique d'une séquence et la qualité de ladite séquence.

Dans un mode d'exécution, le taux prédéfini est au moins supérieur à 50%. Dans un autre mode d'exécution, le taux prédéfini peut être paramétré par l'opérateur, par exemple par une interface utilisateur du dispositif.

Dans un mode d'exécution, l'étape d'enregistrement automatique comprend la mise en mémoire tampon des images échographiques reçues en continu ainsi que des indices de qualités générés associées auxdites images. De cette manière, lorsqu'une séquence vidéo remplissant les critères énoncés ci-dessus est détectée, les images appartenant à cette séquence peuvent être transférées de la mémoire tampon vers une autre mémoire, et/ou regroupées dans un même fichier pour générer une séquence vidéo.

Par souci de clarté, on appellera dans la suite de cette description, la génération et l'enregistrement d'une telle séquence la « validation » d'une telle séquence.

### INDICATEUR TEMPOREL

Dans un mode d'exécution, le procédé comprend une étape de génération et d'affichage en temps réel d'un indicateur temporel 12.

L'indicateur temporel 12 affiché est représentatif du nombre d'images restantes à recevoir ou à classifier pour atteindre une séquence d'un nombre prédéfinie d'images reçues comprenant un taux d'images associées à une même classe supérieur à un taux prédéfini.

Cet indicateur permet avantageusement à l'opérateur de visualiser le temps qu'il lui reste pour valider une séquence vidéo d'une vue particulière du coeur. Cet indicateur permet avantageusement à l'opérateur de visualiser le temps restant durant lequel il doit conserver une image échographique de qualité suffisante, ladite qualité lui étant affichée par l'indicateur de qualité 13 et/ou l'indicateur de confiance.

L'indicateur temporel 12 peut comprendre une valeur numérique tel un décompte. L'indicateur temporel 12 peut comprendre une jauge se remplissant ou se vidant en fonction du temps restant pour valider une séquence vidéo.

Dans un mode d'exécution, le procédé comprend, lorsqu'une première image est classée dans une classe représentant une vue particulière du coeur, la génération et l'affichage automatique de l'indicateur temporel 12.

À chaque nouvelle image classifiée, l'indicateur temporel 12 est préférentiellement mis à jour en temps réel :
- si le nombre d'images classifiées depuis la première image comprend un taux d'images associées à la même classe qui est supérieure au taux prédéfini, alors l'indicateur temporel 12 est mis à jour en indiquant le temps restant ou le nombre d'images restantes à recevoir pour atteindre le nombre d'images prédéfinies et l'enregistrement de la séquence 20 vidéo ;
- si le nombre d'images classifiées depuis la première image comprend un taux d'images associées à la même classe qui est inférieure au taux prédéfini, alors l'indicateur temporel 12 est mis à jour pour indiquer l'échec de la validation, par exemple par la remise à zéro du compteur ou de la jauge ou encore par la disparition de l'affichage d'un tel compteur ou d'une telle jauge.

Le procédé comprend l'affichage en temps réel des images échographiques reçues, de l'indicateur de qualité 13, et optionnellement de l'indicateur temporel 12. Un tel affichage est décrit ci-dessous en référence à la figure 2. L'image affichée par l'afficheur AFF comprend l'image échographique 11 reçue en temps réel ou prise en temps réel par la sonde échographique SECH, l'indicateur de qualité 13 et l'indicateur temporel 12.

### PROGRAMME

Selon un mode d'exécution, le procédé selon l'invention comprend la sélection d'une première classe parmi les classes représentant une vue particulière du coeur telle qu'une des vues mentionnées ci-avant.

La sélection de la première classe génère l'affichage sur l'afficheur AFF d'une première image échographique 15 du coeur préenregistré représentant une telle vue. Préférentiellement, le label 17 pouvant comprendre le nom de ladite vue particulière est également affiché sur l'afficheur AFF. La sélection de la première classe génère également l'affichage d'une première image de consigne 14. La première image de consigne 14 est préférentiellement préenregistrée. L'image de consigne illustre une consigne de position et/ou d'orientation de la sonde échographique SECH sur un patient pour l'obtention de ladite vue correspondant à la classe sélectionnée. L'opérateur est ainsi avantageusement guidé et assisté pour la prise d'une telle vue. Il peut ainsi, sur le même afficheur AFF, visualiser un exemple de la vue qu'il doit prendre, la position et l'orientation de la sonde pour y parvenir. Enfin, l'opérateur peut visualiser en temps réel si la vue qui est acquise est de suffisamment bonne qualité par l'indicateur de qualité 13, et peut avantageusement visualiser l'influence des mouvements qu'il réalise sur la qualité de l'image acquise grâce à l'indicateur de confiance.

Enfin, une fois que l'opérateur a trouvé une image de qualité satisfaisante, il est assisté en temps réel par l'indicateur temporel 12 représentatif du temps pendant lequel il doit maintenir la sonde pour acquérir des images de qualité suffisante. L'opérateur est également assuré, en visualisant l'indicateur temporel 12, qu'une première séquence 20 va être enregistrée en maintenant sa position.

Une fois la première séquence enregistrée ou validée, le procédé peut comprendre la sélection automatique d'une seconde classe parmi les classes représentant une vue particulière du coeur tel qu'une des vues mentionnées ci-avant. Une fois encore, la sélection de ladite seconde classe génère automatiquement l'affichage d'une seconde image de consigne préenregistrée et l'affichage d'une seconde image échographique préenregistrant illustrant une vue échographique.

Selon un mode d'exécution, le procédé comprend en outre la génération et l'affichage d'un indicateur d'avancement 18. L'indicateur d'avancement peut être représentatif du nombre de séquences qui ont été enregistrées. Par exemple, l'indicateur d'avancement est représentatif du nombre de classes représentant une vue particulière du coeur pour laquelle une séquence 20 d'image a été générée et/ou enregistrée automatiquement selon le procédé de l'invention.

Selon un mode d'exécution, le procédé comprend l'affichage de la séquence enregistrée et comprend en outre une seconde validation manuelle de l'opérateur après l'affichage de ladite séquence.

### DISPOSITIF

Selon un aspect, le dispositif selon l'invention comprend des moyens logiciel et matériel pour mettre en oeuvre le procédé tel que décrit ci-dessus.

Un mode de réalisation du dispositif selon l'invention est décrit maintenant en référence à la figure 2.

Le dispositif comprend un récepteur REC. Le récepteur REC est destiné à être connecté à une sonde échographique SECH de manière à recevoir en continu et en temps réel des images échographiques 11 acquises par ladite sonde échographique SECH.

Le récepteur REC peut être connecté à la sonde échographique SECH par une liaison filaire ou une liaison sans-fil, par exemple par une connexion Bluetooth ou une connexion WI-FI ou tout autre protocole d'échanges de données connu de l'homme de l'art.

Le récepteur REC peut comprendre ou être associé à une ou plusieurs mémoire(s) pour stocker temporairement les images reçues. Le récepteur REC est directement ou indirectement connecté à l'afficheur AFF pour transmettre à l'afficheur AFF les images échographiques acquises 11.

Le dispositif comprend en outre des moyens pour mettre en oeuvre un classifieur CLASS tel que décrit ci-avant. Le classifieur est configuré pour recevoir les images échographiques 11, associer en temps réel une classe 14 aux images échographiques, et générer en temps réel l'indicateur de qualité 13. Le classifieur est directement ou indirectement connecté à l'afficheur AFF pour transmettre à l'afficheur AFF les indicateurs générés.

Selon une alternative, le classifieur est mis en oeuvre par un équipement électronique distant, tel qu'un serveur distant. Dans ce cas de figure, le dispositif comprend une interface pour échanger des données avec l'équipement distant afin de transmettre des données et récupérer le résultat des données traitées, c'est-à-dire classifiées.

Enfin, dans la présente invention, il est entendu que lorsque la classification est mise en oeuvre totalement en partie par un équipement distant, le dispositif de l'invention peut s'interpréter comme étant le système comportant d'une part le dispositif local décrit dans la présente demande et les moyens distants permettant de mettre en oeuvre la fonction de classification.

Le dispositif comprend en outre au moins un processeur ou un calculateur CALC associé à une mémoire pour exécuter au moins une partie des étapes du procédé selon l'invention. Par exemple, ledit processeur peut être configuré pour exécuter les étapes de classification 200, de génération d'affichage de l'indicateur de qualité 300 et de l'indicateur temporel 500 et/ou d'enregistrement automatique 300 de la séquence 20 d'images. Ledit processeur peut être connecté à l'afficheur AFF pour transmettre à l'afficheur AFF l'indicateur temporel 12.

Le dispositif 1 peut également comprendre une pluralité de processeurs, associés chacun à une ou plusieurs mémoires, et configurés pour ensemble exécuter de telles étapes. Dans un mode de réalisation, le ou les processeurs peuvent être déportés et connectés à l'afficheur par un réseau de données.

Le dispositif comprend en outre une mémoire pour stocker ou enregistrer les séquences générées par le procédé selon l'invention. Dans un mode de réalisation, le dispositif comprend en outre un émetteur EMM connectée à ladite mémoire MEM pour transmettre lesdites séquences enregistrées sur ladite mémoire MEM vers un réseau de données.

L'afficheur AFF peut comprendre des moyens pour réceptionner les différentes informations 11, 13, 21, 12 reçues par les différents moyens REC, CLASS, PROC du dispositif pour générer l'image finale à afficher.

## Revendications

1. Un dispositif (1) d'assistance à la création d'une séquence d'images échographiques, configuré pour recevoir en temps réel des images échographiques (11) acquises par une sonde échographique (SECH) connectée audit dispositif (1), et comprenant :
▪ un premier calculateur, associé à une première mémoire, pour mettre en oeuvre un classifieur (CLASS) configuré pour classifier en temps réel les images échographiques reçues (11) et associer à chaque image une classe (14) pour générer un indicateur de qualité (13) en fonction de ladite classe (14) associée ;
▪ un afficheur (AFF) pour, en temps réel, afficher le dernier indicateur de qualité (13) généré ;
▪ un second calculateur (CALC), associé à une seconde mémoire, et configuré pour exécuter les étapes suivantes :
▪ lorsqu'une séquence d'un nombre prédéfini d'images reçues comprend un taux d'images associées à une même classe supérieur à un taux prédéfini, l'enregistrement automatiquement de ladite séquence d'images (20) dans une troisième mémoire (MEM) ; et
▪ la génération et l'affichage sur l'afficheur en temps réel d'un indicateur temporel (12) représentant le nombre d'images restantes à recevoir pour atteindre ladite séquence d'un nombre prédéfini d'images reçues comprenant un taux d'images associées à une même classe supérieur à un taux prédéfini.

2. Dispositif selon la revendication 1, dans lequel le classifieur (CLASS) est configuré pour classifier une image parmi :
▪ une pluralité de classes, représentant chacune une vue particulière de l'organe du coeur ; et
▪ au moins une classe représentant une vue de qualité insuffisante.

3. Dispositif selon la revendication 2, dans lequel le classifieur (CLASS) est mis en oeuvre au moyen d'une fonction apprenante configurée à partir d'un apprentissage automatique supervisé.

4. Dispositif selon la revendication précédente dans lequel le classifieur (CLASS) est mis en oeuvre au moyen d'un réseau de neurones entrainé par une série d'images échographiques du coeur labélisées, lesdits labels comportant une information caractéristique :
• d'une prise de vue d'une image d'un organe comportant un angle de vue et/ou une taille caractéristique ;
• d'une qualité d'image ;
• de la présence d'une portion d'une image du coeur ; et/ou
• de la présence d'un contour de forme anatomique mesurable.

5. Dispositif (1) selon l'une des revendications précédentes, dans lequel l'indicateur de qualité (13) affiché comprend un indicateur coloré dont la couleur dépend de la classe associée à la dernière image classifiée.

6. Dispositif (1) selon la revendication 5, dans lequel au moins une des dimensions de l'indicateur coloré affiché dépend de ladite valeur de confiance.

7. Dispositif (1) selon l'une quelconque des revendications précédentes, dans lequel l'indicateur temporel (12) affiché comprend une jauge dont le remplissage dépend de la valeur de l'indicateur temporel.

8. Dispositif (1) selon l'une quelconque des revendications précédentes dans lequel l'afficheur est conçu pour afficher, en temps réel, les images échographiques reçues (11).

9. Système (2) comprenant un dispositif (1) selon l'une quelconque des revendications 1 à 8 et une sonde échographique (SECH) apte à être connectée audit dispositif (1) de manière à transmettre les images échographiques captées par la sonde au dispositif.

10. Procédé (1000) d'assistance à la génération d'une séquence d'images échographiques mise en oeuvre par ordinateur comprenant :
▪ la réception (100) d'images échographiques (11) depuis une sonde échographique (SEC) ;
▪ la classification (200) en temps réel des images échographiques reçues (11), et l'association à chaque image d'une classe ;
▪ la génération et l'affichage (300) d'un indicateur de qualité (13) en fonction de ladite classe (14) associée à l'image échographique classifiée ;
▪ lorsqu'une séquence d'un nombre prédéfini d'images reçues comprend un taux d'images associées à une même classe supérieur à un taux prédéfini, l'enregistrement automatiquement (400) de ladite séquence d'images (20) dans une mémoire (MEM) ;
▪ la génération et l'affichage (500) en temps réel d'un indicateur temporel (12) représentant le nombre d'images restantes à recevoir pour atteindre ladite séquence d'un nombre prédéfini d'images reçues comprenant un taux d'images associées à une même classe supérieur à un taux prédéfini.

11. Procédé selon la revendication précédente, dans lequel une fraction prédéfinie des images reçues sont classifiées, ladite fraction prédéfinie étant pilotable par une interface utilisateur.

12. Procédé selon l'une des revendications 10 ou 11, dans lequel les images échographiques reçues (11) sont affichées en continu.

13. Procédé selon l'une des revendications 10 à 12, dans lequel le procédé comprend en outre la sélection d'une première classe représentant une première vue particulière du coeur, ladite sélection générant automatiquement :
▪ l'affichage d'une première image préenregistrée correspondant à une image de la première vue échographique du coeur ladite première classe sélectionnée, et/ou
▪ l'affichage d'une première consigne préenregistrée de position de sonde échographique sur un patient pour capter ladite première vue particulière du coeur.

14. Produit programme d'ordinateur comprenant des instructions qui conduisent un dispositif à exécuter les étapes du procédé selon l'une des revendications 10 à 13.

15. Support lisible par ordinateur, sur lequel est enregistré le programme d'ordinateur selon la revendication 14.

## Patentansprüche

1. Vorrichtung (1) zur Unterstützung bei der Erstellung einer Ultraschallbildsequenz, die so ausgelegt ist, dass sie in Echtzeit Ultraschallbilder (11) empfängt, die von einer an dieser Vorrichtung (1) angeschlossenen Ultraschallsonde (SECH) erfasst werden, und umfassend:
▪ einen ersten Rechner, der einem ersten Speicher zugeordnet ist, um einen Klassifizierer (CLASS) zu implementieren, der so ausgelegt ist, dass er die empfangenen Ultraschallbilder (11) in Echtzeit klassifiziert und jedem Bild eine Klasse (14) zuordnet, um einen Qualitätsindikator (13) in Abhängigkeit von der zugeordneten Klasse (14) zu generieren;
▪ eine Anzeigevorrichtung (AFF), um in Echtzeit den zuletzt generierten Qualitätsindikator (13) anzuzeigen;
▪ einen zweiten Rechner (CALC), der einem zweiten Speicher zugeordnet und so ausgelegt ist, dass er die folgenden Schritte ausführt:
▪ wenn eine Sequenz einer vordefinierten Anzahl empfangener Bilder eine Bildrate umfasst, die einer gleichen Klasse zugeordnet ist, die größer ist als eine vordefinierte Rate, das automatische Speichern der Bildsequenz (20) in einem dritten Speicher (MEM); und
▪ das Generieren und das Anzeigen auf der Anzeigevorrichtung in Echtzeit eines Zeitindikators (12), der die Anzahl der verbleibenden Bilder darstellt, die empfangen werden müssen, um die Sequenz einer vordefinierten Anzahl empfangener Bilder zu erreichen, die eine Bildrate umfasst, die einer gleichen Klasse zugeordnet ist, die größer ist als eine vordefinierten Rate.

2. Vorrichtung nach Anspruch 1, wobei der Klassifizierer (CLASS) ausgelegt ist, um ein Bild zu klassifizieren in:
▪ eine Vielzahl von Klassen, die jeweils eine bestimmte Ansicht des Herzorgans repräsentieren; und
▪ mindestens eine Klasse, die eine Ansicht in unzureichender Qualität darstellt.

3. Vorrichtung nach Anspruch 2, wobei der Klassifizierer (CLASS) mittels einer Lernfunktion implementiert wird, die auf der Basis eines überwachten maschinellen Lernens konfiguriert ist.

4. Vorrichtung nach vorhergehendem Anspruch, wobei der Klassifizierer (CLASS) mittels eines Neuronennetzwerks implementiert wird, das von einer Reihe gekennzeichneter Ultraschallbilder des Herzens trainiert wird, wobei die Kennzeichnungen eine charakteristische Information aufweisen:
• einer Aufnahme eines Bildes eines Organs, die einen charakteristischen Blickwinkel und/oder eine charakteristische Größe aufweist;
• einer Bildqualität;
• des Vorhandenseins eines Teils eines Bildes des Herzens; und/oder
• des Vorhandenseins einer Kontur in einer messbaren anatomischen Form.

5. Vorrichtung (1) nach einem der vorhergehenden Ansprüche, wobei der angezeigte Qualitätsindikator (13) einen farbigen Indikator umfasst, dessen Farbe von der Klasse abhängt, die dem zuletzt klassifizierten Bild zugeordnet ist.

6. Vorrichtung (1) nach Anspruch 5, wobei wenigstens eine der Abmessungen des angezeigten farbigen Indikators vom Vertrauenswert abhängt.

7. Vorrichtung (1) nach einem der vorhergehenden Ansprüche, wobei der angezeigte Zeitindikator (12) eine Messlehre umfasst, deren Füllung vom Wert des Zeitindikators abhängt.

8. Vorrichtung (1) nach einem der vorhergehenden Ansprüche, wobei die Anzeigevorrichtung zum Anzeigen der empfangenen Ultraschallbilder (11) in Echtzeit ausgebildet ist.

9. System (2), das eine Vorrichtung (1) nach einem der Ansprüche 1 bis 8 und eine Ultraschallsonde (SECH) umfasst, die an diese Vorrichtung (1) angeschlossen werden kann, um die von der Sonde aufgenommenen Ultraschallbilder an die Vorrichtung zu übertragen.

10. Verfahren (1000) zur Unterstützung des Generierens einer computergestützten Ultraschallbildsequenz, umfassend:
▪ das Empfangen (100) von Ultraschallbildern (11) von einer Ultraschallsonde (SEC);
▪ das Klassifizieren (200) in Echtzeit der empfangenen Ultraschallbilder (11) und das Zuordnen einer Klasse zu jedem Bild;
▪ das Generieren und Anzeigen (300) eines Qualitätsindikators (13) in Abhängigkeit von dieser Klasse (14), die dem klassifizierten Ultraschallbild zugeordnet wurde;
▪ wenn eine Sequenz einer vordefinierten Anzahl empfangener Bilder eine Bildrate umfasst, die einer gleichen Klasse zugeordnet ist, die größer ist als eine vordefinierte Rate, das automatische Speichern (400) der Bildsequenz (20) in einem Speicher (MEM);
▪ das Generieren und das Anzeigen (500) in Echtzeit eines Zeitindikators (12), der die Anzahl der verbleibenden Bilder darstellt, die empfangen werden müssen, um die Sequenz einer vordefinierten Anzahl empfangener Bilder zu erreichen, die eine Bildrate umfasst, die einer gleichen Klasse zugeordnet ist, die größer ist als eine vordefinierten Rate.

11. Verfahren nach vorhergehendem Anspruch, wobei ein vordefinierter Bruchteil der empfangenen Bilder klassifiziert wird, wobei der vordefinierte Bruchteil über eine Benutzerschnittstelle steuerbar ist.

12. Verfahren nach einem der Ansprüche 10 oder 11, wobei die empfangenen Ultraschallbilder (11) kontinuierlich angezeigt werden.

13. Verfahren nach einem der Ansprüche 10 bis 12, wobei das Verfahren ferner die Auswahl einer ersten Klasse umfasst, die eine erste besondere Ansicht des Herzens darstellt, wobei die Auswahl automatisch generiert:
▪ die Anzeige eines ersten voraufgezeichneten Bildes, das einem Bild der ersten Ultraschallansicht des Herzens der ersten ausgewählten Klasse entspricht, und/oder
▪ die Anzeige eines ersten voraufgezeichneten Sollwerts für die Position der Ultraschallsonde an einem Patienten zur Erfassung der ersten besonderen Ansicht des Herzens.

14. Rechnerprogrammprodukt, das Anweisungen umfasst, die eine Vorrichtung anleiten, die Schritte des Verfahrens nach einem der Ansprüche 10 bis 13 auszuführen.

15. Rechnerlesbares Medium, auf dem das Rechnerprogramm nach Anspruch 14 gespeichert ist.

## Claims

1. A device (1) for assisting in creating a sequence of ultrasound images of the heart, configured to receive in real time ultrasound images (11) acquired by an ultrasound probe (SECH) connected to said device (1), and comprising:
▪ a first calculator, associated with a first memory, for implementing a classifier (CLASS) configured to classify in real time the received ultrasound images (11) and to associate each image with a class (14) for generating a quality indicator (13) as a function of said associated class (14) ;
▪ a display (AFF) for, in real time, displaying the last generated quality indicator (13);
▪ a second calculator (CALC), associated with a second memory, and configured to perform the following steps of:
▪ when a sequence of a predefined number of received images includes a rate of images associated with a same class greater than a predefined rate, automatically recording said sequence of images (20) in a third memory (MEM); and
▪ generating and displaying, on the display in real time, a time indicator (12) representing the number of remaining images to be received to reach said sequence of a predefined number of received images including a rate of images associated with a same class greater than a predefined rate.

2. The device according to claim 1, wherein the classifier (CLASS) is configured to classify an image from among:
▪ a plurality of classes, each representing a particular view of the heart organ; and
▪ at least one class representing a view of insufficient quality.

3. The device according to claim 2, wherein the classifier (CLASS) is implemented by means of a learning function configured from supervised machine learning.

4. The device according to the preceding claim, wherein the classifier (CLASS) is implemented by means of a neural network trained by a series of labelled ultrasound images of the heart, said labels including characteristic information of:
• a shot of an image of an organ including a characteristic angle of view and/or size;
• an image quality; and
• the presence of a portion of an image of the heart; and/or
• the presence of a measurable anatomical shape contour.

5. The device (1) according to one of the preceding claims, wherein the displayed quality indicator (13) comprises a colored indicator whose color depends on the class associated with the last classified image.

6. The device (1) according to claim 5, wherein at least one of the dimensions of the displayed colored indicator depends on said confidence value.

7. The device (1) according to any of the preceding claims, wherein the displayed time indicator (12) comprises a gauge whose filling depends on the value of the time indicator.

8. The device (1) according to any of the preceding claims, wherein the display is configured for displaying, in real time, the received ultrasound images (11).

9. A system (2) comprising a device (1) according to any of claims 1 to 8 and an ultrasound probe (SECH) capable of being connected to said device (1) so as to transmit ultrasound images captured by the probe to the device.

10. A method (1000) for assisting in generating a computer-implemented sequence of ultrasound images comprising:
▪ receiving (100) ultrasound images (11) from an ultrasound probe (SEC);
▪ classifying (200) in real time the received ultrasound images (11), and associating each image with a class;
▪ generating and displaying (300) a quality indicator (13) as a function of said class (14) associated with the classified ultrasound image;
▪ when a sequence of a predefined number of received images includes a rate of images associated with a same class greater than a predefined rate, automatically recording (400) said sequence of images (20) in a memory (MEM);
▪ generating and displaying (500) in real time a time indicator (12) representing the number of remaining images to be received to reach said sequence of a predefined number of received images including a rate of images associated with a same class greater than a predefined rate.

11. The method according to the preceding claim, wherein a predefined fraction of the received images are classified, said predefined fraction being controllable by a user interface.

12. The method according to one of claims 10 or 11, wherein the received ultrasound images (11) are continuously displayed.

13. The method according to one of claims 10 to 12, wherein the method further comprises selecting a first class representing a particular first view of the heart, said selection automatically generating:
▪ displaying a first pre-recorded image corresponding to an image of the first ultrasound view of the heart of said first selected class, and/or
▪ displaying a first pre-recorded setpoint of ultrasound probe position on a patient to capture said first particular view of the heart.

14. A computer program product comprising instructions that cause a device to perform the steps of the method according to one of the claims 10 to 13.

15. A computer readable support, on which the computer program according to claim 14 is recorded.
